# EUROPEAN PATENT SPECIFICATION

(11) **EP 1 806 134 B1**
(45) Date of publication and mention of the grant of the patent: **11.07.2012**
(21) Application number: 05785845.8
(22) Date of filing: 16.09.2005
(51) Int. Cl.: A61K 31/198, A61K 31/7068, A61K 31/7072, A61K 31/7076, A61K 31/708, A61P 1/04, A61P 1/14, A61P 43/00, A23L 1/30, A23L 1/305, C07H 19/10, C07H 19/20, A23L 1/229, A61K 31/7088

(54) **AGENT AND FOOD FOR PREVENTING/IMPROVING FUNCTIONAL DIGESTIVE DISORDER**
MITTEL UND NAHRUNGSMITTEL ZUR PRÄVENTION/VERBESSERUNG VON FUNKTIONALEN VERDAUUNGSSTÖRUNGEN
AGENT ET ALIMENT POUR LA PREVENTION/L'AMELIORATION DU TROUBLE DIGESTIF FONCTIONNEL

(30) Priority: 17.09.2004 JP 2004271884
(43) Date of publication of application: 11.07.2007
(60) Divisional application: 09005515.3
(73) Proprietor: AJINOMOTO CO., INC., Chuo-ku Tokyo 104-8315 (JP)
(72) Inventor: UNEYAMA, Hisayuki, Ajinomoto Co., Inc., Kawasaki-shi, Kanagawa 2108681 (JP); TANAKA, Tatsuro, Ajinomoto Co., Inc., Kawasaki-shi, Kanagawa 2108681 (JP); TORII, Kunio, Kawasaki-shi, Kanagawa 2108681 (JP); FUJITA, Shinichi, Kawasaki-shi, Kanagawa 2108681 (JP)
(74) Representative: Nicholls, Kathryn Margaret
(86) International application number: PCT/JP2005/017548
(87) International publication number: WO 2006/030980

(56) References cited:
- EP-A- 1 767 201
- JP-A- 06 038 703
- JP-A- 2001 314 172
- JP-A- 2003 524 636
- JP-A- 2004 521 898
- DATABASE BIOSIS [Online] BIOSCIENCES INFORMATION SERVICE, PHILADELPHIA, PA, US; 1995, BURNS G A ET AL: "Expression of mRNA for the N-methyl-D-aspartate (NMDAR1) receptor and vasoactive intestinal polypeptide (VIP) co-exist in enteric neurons of the rat" XP002447633 Database accession no. PREV199698567050 & JOURNAL OF THE AUTONOMIC NERVOUS SYSTEM, vol. 55, no. 3, 1995, pages 207-210, ISSN: 0165-1838
- KIRCHGESSNER A L: "Glutamate in the enteric nervous system" CURRENT OPINION IN PHARMACOLOGY, vol. 1, 2001, pages 591-596, XP002447628
- KESSLER J-P ET AL: "Evidence that activation of N-methyl-D-aspartate (NMDA) and non-NMDA receptors within the nucleus tractus solitarii triggers swallowing" EUROPEAN JOURNAL OF PHARMACOLOGY, vol. 201, 1991, pages 59-67, XP002447629
- BECQUET D ET AL: "Glutamate, GABA, glycine and taurine modulate serotonin synthesis and release in rostral and caudal rhombencephalic raphe cells in primary cultures" NEUROCHEMSITRY INTERNATIONAL, vol. 23, no. 3, 1993, pages 269-283, XP002447630
- ONOUE S ET AL: "Pituitary adenylate cyclose-activating polypeptide and vasoactive intestinal peptide attenuate glutamate-induced nNOS activation and cytotoxicity" REGULATORY PEPTIDES, vol. 107, 2002, pages 43-47, XP002447631
- UNEYAMA T. ET AL: 'Meiso Shinkei ni Okeru Monosodium Glutamate Otosei' THE JAPANESE JOURNAL OF TASTE AND SMELL RESEARCH vol. 6, no. 3, 1999, pages 645 - 648, XP002999856
- HORNBY P.J.: 'Receptors and transmission in the brain-gut axis. II. Excitatory amino acid receptors in the brain-gut axis' AM.J.PHYSIOL.GASTROINTEST. LIVER PHYSIOL. vol. 280, no. 6, 2001, pages G1055 - G1060, XP002999857

## Description

### Technical Field

The present invention relates to an agent for use in the prophylaxis or improvement of a functional gastrointestinal disorder. More particularly, the present invention relates to an agent for use in the prophylaxis or improvement of functional gastrointestinal disorders (FGIDs), particularly, upper gastrointestinal dysfunctions such as functional dyspepsia (FD) (e.g., abdominal pain, heavy stomach, or heartburn), and gastroesophageal reflux disease (GERD); a gastrointestinal motility function promoter; or as an agent for the prophylaxis or improvement of dysphagia. The present invention also relates to a food for the prophylaxis or improvement of a functional gastrointestinal disorder.

### Background Art

Even with the advancement in endoscopic diagnosis, there are many cases where a complaint of the upper gastrointestinal symptoms such as upper abdominal pain, discomfort, postprandial heavy stomach, nausea, or vomiting cannot be fully explained. Such condition, where a gastrointestinal symptom is reported but no organic disease is found by a general checkup including endoscopic examination, and no finding to elucidate the symptom is available is referred to as an FD (functional dyspepsia: non-ulcer dyspepsia (NUD): upper abdominal indefinite complaint). According to The American Gastroenterological Association, FD is defined to be a pathology where organic diseases such as peptic ulcer and cancer symptoms are not observed, but upper abdominal indefinite complaint continues for 4 weeks or longer, such as feeling of fullness in the abdomen, nausea/vomiting, upper abdominal pain, anorexia, or abnormal bowel movement, based on the retention of contents in the stomach.

On the other hand, in Japan, such case has been determined to be "upper abdomen gastrointestinal complaint associated with chronic gastritis" irrespective of organic findings, and, in clinical situations, diagnosed conventionally as "gastritis" or "chronic gastritis". Currently, the subtype of FD includes an ulcer symptom type, a gastrointestinal dysmotility type and non-specific type, which include conventional gastroatonia, nervous dyspepsia and gastric neurosis.

Even in cases where an organic disease (reflux esophagitis, peptic ulcer, acute gastritis, gastrointestinal cancer, pancreas/biliary disease etc.) is clearly observed, abdominal pain, discomfort, postprandial heavy stomach, nausea·vomiting and the like are also found. Accordingly, there is an urgent need for the improvement of such discomfortable feeling to ensure better QOL of patients. When NUD is joined with lower abdomen indefinite complaint such as defecation difficulty, unrelieved feeling after defecation, abdominal pain, or feeling of fullness in the abdomen, due to constipation, about 30%-50% of the total population of Japan is assumed to have experienced some gastrointestinal indefinite complaint. The development of abdominal indefinite complaint is considered to be influenced by sex, aging, stress or overweight due to the western style diet, and is a disease representing the modern society along with the lifestyle-related diseases. Even though it is such a serious disease, the etiology of the gastrointestinal indefinite complaint is merely suggested to involve various diseases (chronic gastritis, diabetes, overweight, constipation etc.), and the only suggested mechanism of its onset is degraded gastrointestinal motility function.

In addition, many of the patients with a progressive degenerative disease of the brain such as Parkinson's disease, Huntington chorea or olivopontocerebellar atrophy, or cerebral apoplexy, also develop gastrointestinal motility dysfunction, and improvement of QOL by the improvement of gastrointestinal motility function is considered to be necessary. It is considered that many of these patients cannot report indefinite complaint by themselves, e.g. due to logopathy or disturbance of consciousness. Thus, a treatment that removes a disturbance of sensation such as indefinite complaint simultaneously with a treatment of organic dysfunction leads to the improvement of QOL in a true sense.

5-HT4 receptor agonists have heretofore been used for the treatment of FD. For example, cisapride and metoclopramide have a hyperanakinesia action on the stomach and intestines, and have been used for the treatment of the symptoms and the like of chronic gastritis, feeling of fullness in the abdomen, reflux esophagitis, abdominal indefinite complaint and pseudoileus. However, metoclopramide shows a side effect of extrapyramidal symptoms caused by the action on dopamine D2 receptors in the central nervous system, and cisapride has also been clarified to show parkinsonian symptoms. While mosapride etc. have also been used, the effect is not always sufficient, and side effects such as feeling of fullness in the abdomen appear. While H2 antagonists and proton pump inhibitors have been used for the treatment of gastroesophageal reflux disease (GERD), since the safety of long-term administration has not been established, a periodic examination is necessary. Therefore, it is difficult to expect a treatment effect of these existing pharmaceutical agents while ensuring sufficient safety.

Moreover, therapeutic drugs for FD and gastrointestinal indefinite complaint accompanying a gastrointestinal organic disorder, partial 5-HT3 receptor agonist, nitroglycerol, nitric oxide (hereinafter NO)-releasing drugs such as nitrate etc., are known. However, since 5-HT receptors and nitrergic proteins, which are the target of these pharmaceutical agents, are distributed in not only the gastrointestinal mucous membrane but also organs in the body including brain, the agents show various physiological activities. To be specific, when a 5-HT3 antagonist is used as an antiemetic agent, a non-specific 5-HT3 receptor agonist particularly induces nausea and vomiting. It is also known that nonspecific NO release in the systemic circulation induces low blood pressure. Therefore, it is necessary to develop a safe and highly effective pharmaceutical agent that can act on these targets only in the gastrointestinal organs.

On the other hand, glutamine has been reported to show an effect of improving organic gastrointestinal diseases, such as ulcer, without expressing a side effect (Elia M, Lunn PG., Nutrition. 1997 Jul-Aug; 13(7-8): 743-7). However, glutamine has low solubility, is highly unstable in an aqueous solution, and lacks convenience. A report has documented that addition of monosodium glutamate alone, and both glutamic acid and sodium inosinate, to diet can enhance gastric secretion in an experiment model of atrophy gastritis, which is also one of the organic diseases (Vasilevskaia LS, et al., Vopr Pitan. 1993 May-Jun; (3): 29-33, Rymshina MV & Vasilevskaia LS., Vopr Pitan. 1996; (1): 9-11). Monosodium glutamate has been confirmed to enhance gastric secretion in atrophy gastritis patients, and moreover, glutamic acid and sodium inosinate show a potential to be digestion promoters in atrophy gastritis patients (Kochetkov AM, Vopr Pitan. 1992 Sep-Dec;(5-6): 19-22, Shlygin GK, Klin Med (Mosk). 1991 Aug; 69(8): 66-70). However, a treatment effect of glutamic acid and sodium inosinate has not been suggested yet for the sensory abnormality (indefinite complaint) associated with NUD and digestive trouble.

In addition, there are reports on several attempts to improve gastrointestinal function and increase efficiency of nutrition support in patients with organic disorder in the gastrointestinal tract or patients with lower function caused thereby, by enteral administration of a glutamic acid-containing composition for the purpose of protecting the mucous membrane and improving the motility of the lower gastrointestinal tract (DE-B-4133366, US patent application publication No. 2003/138476, EP-B-0318446, JP-A-56-57385, CA-B-2404005, JP-A-48-30583). In these cases, however, maintenance of gastrointestinal barrier by, for example, protection of the mucous membrane and improvement of the motility of the lower gastrointestinal tract are desired by addition of glutamic acid to a nutritional composition such as amino acid and protein, and a treatment effect on FD is not suggested.

### Disclosure of the Invention

As mentioned above, 5-HT agonists and NO releasing agents have conventionally been used as therapeutic agents for FD. While serotonin and NO are systemically distributed, they are particularly abundantly present in the gastrointestinal tract. Particularly, it is considered that about 80% of serotonin is present in the gastrointestinal mucosal epithelium. There are abundant findings relating to the physiological activities of NO and serotonin in the gastrointestinal tract. NO in the gastrointestinal mucous membrane is considered to relate to receptive relaxation upon food intake, promotion of mucus secretion, repair of disordered mucous membrane, enhancement of gastrointestinal immunity, sterilization of gastrointestinal lumen, improvement of microcirculation by increased mucosal blood flow and to antiplatelet aggregation effect, and plays an important role for the maintenance of gastrointestinal function. In addition, serotonin is a main physiologically active substance of the gastrointestinal tract, which is responsible for gastrointestinal motility regulation, gastrointestinal exocrine regulation (gastric-acid secretion, pancreatic exocrine secretion etc.). When the action of these substances is artificially inhibited, the gastrointestinal function is prevented, which causes ulcer, gastrointestinal bleeding, and abnormal motility.

Therefore, a gastrointestinal indefinite complaint can be taken as a warning from the gastrointestinal tract, and it is easily assumed that a gastrointestinal indefinite complaint can be caused by a nonorganic gastrointestinal dysfunction of a mild level which is pathologically difficult to judge, not to mention an event of gastrointestinal organic pathology. Therefore, 5-HT agonists that promote serotonin release and NO releasing agents that promote NO have conventionally been used. However, these pharmaceutical agents are problematic in terms of systemic side effects and safety as mentioned above. Hence, there is a demand for the development of a pharmaceutical agent that specifically promotes release of serotonin and NO in the gastrointestinal tract and improves various conditions associated with FD.

The present invention has been made in this situation and aims at providing a pharmaceutical agent or a food capable of improvement of functional gastrointestinal disorders, particularly upper gastrointestinal dysfunction such as functional dyspepsia or esophageal reflux, promotion of gastrointestinal motility function, and prophylaxis or improvement of dysphagia.

The present inventors have conducted intensive studies in an attempt to solve the above-mentioned problems and found that when a basic amino acid salt of glutamic acid is administered to a subject, the concentration of NO and serotonin increases only in the gastrointestinal tract to promote gastrointestinal motility function, and therefore, functional gastrointestinal disorders and dysphagia can be improved without inducing systemic side effects, which resulted in the completion of the present invention.

Here, with regard to the absorbability of monosodium glutamate, it has been reported that when monosodium glutamate is taken orally, not less than 90% of glutamic acid taken is completely oxidized on the gastrointestinal mucous membrane into carbon dioxide and water and the remaining 10% or less is converted to alanine and lactic acid, and thus, the amount of glutamic acid that transfers into blood is extremely small (PJ. Reeds, DG Burin, B Stoll, Jahoor, J. Nutrition 130: 978S (2000)). Moreover, the study of Niijima et al. affords the finding that administration of monosodium glutamate into the stomach and duodenum activates the vagus nerve afferent pathway, but intravenous and intraportal administration of monosodium glutamate does not activate the vagus nerve (Niijima A., et al., Physiol Behav. 1991 May; 49(5): 1025-8, Niijima A., et al., J Nutr. 2000 Apr; 130 (4S Suppl): 9715-3S). From these findings, it is considered that even if a part of glutamic acid is absorbed and transferred to the systemic circulation, glutamic acid does not directly activate the vagus nerve afferent pathway in the body.

From these findings, the action mechanism of improvement of functional gastrointestinal disorders by the pharmaceutical agent of the present invention is assumed to be as follows. That is, when the pharmaceutical agent of the present invention is administered to the subject of administration, release of NO and/or serotonin is promoted only in the stomach mucous membrane. As a result, the concentration of NO and/or serotonin increases only in such topical environment, and gastrointestinal motility function is promoted. Thus, the indefinite complaint associated with functional gastrointestinal disorders such as FD and the like can be improved safely and effectively. In addition, since the amount of active ingredient transferred to blood is extremely low, systemic side effects are seldom induced.

The present invention encompasses the following.
(1) An agent for use in the prophylaxis or improvement of a functional gastrointestinal disorder, which comprises a basic amino acid salt of glutamic acid as an active ingredient.
(2) The agent of (1), wherein the basic amino acid is selected from the group consisting of arginine, lysine and ornithine.
(3) The agent of (1), wherein the basic amino acid is arginine.
(4) An agent for use in the prophylaxis or improvement of a functional gastric disorder comprising an arginine salt of glutamic acid as an active ingredient.
(5) The agent of any one of (1) to (4), wherein the aforementioned functional gastrointestinal disorder is upper gastrointestinal dysfunction.
(6) The agent of (5), wherein the aforementioned upper gastrointestinal dysfunction is functional dyspepsia or gastroesophageal reflux disease.
(7) The agent of any one of (1) to (6), wherein a daily dose of the aforementioned active ingredient to an adult is 0.01 g to 20 g.
(8) An agent for promoting gastrointestinal motility function, which comprises, a basic amino acid salt of glutamic acid as an active ingredient.
(9) An agent for the prophylaxis or improvement of dysphagia, which comprises a basic amino acid salt of glutamic acid as an active ingredient.
(10) A food for promoting gastrointestinal motility function, which comprises a basic amino acid salt of glutamic acid.
(11) A food for the prophylaxis or improvement of dysphagia, which comprises a basic amino acid salt of glutamic acid.
(12) An agent according to any of (1) to (7) which is a food.
(13) The food of any one of (10) to (12), wherein the content of the basic amino acid salt of glutamic acid is 0.01 wt% to 10 wt%.
(14) The food of (13), which is a food with health claims or a dietary supplement.
(15) The food of (14), wherein the aforementioned food with health claims is a food for specified health uses or a food with nutrient function claims.
(16) A commercial package comprising a composition comprising a basic amino acid salt of glutamic acid, and written matter stating that the composition can or should be used for at least one of: prophylaxis or improvement of a functional gastrointestinal disorder, promotion of gastrointestinal motility function and prophylaxis or improvement of dysphagia.
(17) A commercial package according to (16) wherein the composition is a food.
(18) Use of a basic amino acid salt of glutamic acid for the production of an agent, food or commercial package according to any one of (1) to (17).

### Brief Description of the Drawings

Fig. 1 shows one example of the study results of promotion of stomach emptying by monosodium glutamate and arginine glutamic acid salt.
Fig. 2 shows one example of the study results of promotion of stomach emptying by lysine glutamic acid salt and calcium glutamate.

### Best Mode for Embodying the Invention

The embodiment of the present invention is explained in the following.

As used herein, the "functional gastrointestinal disorder" refers to a pathology where organic diseases such as peptic ulcer and cancer symptoms are not observed, but upper abdominal indefinite complaint continues such as feeling of fullness in the abdomen, nausea, vomiting, upper abdominal pain, anorexia and abnormal bowel movement, based on the retention of contents in gastrointestinal tract, particularly the stomach. It means a condition without organic disease of the gastrointestinal tract, but with a reproducible gastrointestinal symptom that degrades QOL of patients. The "gastrointestinal tract" in the present invention refers to a series of luminal organs involved in digestion from mouth cavity to anus and, for example, pharynx, esophagus, stomach, small intestine (duodenum, jejunum, ileum) and large intestine can be mentioned. The "upper gastrointestinal tract" refers to pharynx, esophagus, stomach and duodenum.

As used herein, the "functional dyspepsia" refers to a pathology where organic diseases such as peptic ulcer and cancer symptoms are not observed, but upper abdominal indefinite complaint continues such as feeling of fullness in the abdomen, nausea, vomiting, upper abdominal pain, anorexia and abnormal bowel movement, based on the retention of the contents in the stomach. It means a condition without organic disease of the gastrointestinal tract, but with a reproducible gastrointestinal symptom that degrades QOL of patients. The dyspepsia includes diseases so far diagnosed as chronic gastritis and gastritis, and often shows symptoms such as abdominal pain, heavy stomach and heartburn. In recent years, 40-60% of the outpatients of medical practitioners are said to suffer from functional dyspepsia, and Helicobacter pylori removal therapy tends to increase the number of functional dyspepsia.

Furthermore, the "gastroesophageal reflux disease" includes reflux esophagitis and is developed by reflux of gastric acid and shows specific symptoms such as heartburn and flow up of gastric acid to the mouth. Moreover, while "swallowing" means gulping water and food, it is closely related to not only mouth cavity and pharynx, but also motility of gastrointestinal tract such as esophagus, as evidenced by misswallowing and vomiting due to sticking of swallowed food bolus in the esophagus.

An agent for use in the prophylaxis or improvement of a functional gastrointestinal disorder, an agent for the promotion of gastrointestinal motility function and an agent for the prophylaxis or improvement of dysphagia are agents for the prophylaxis or improvement to improve upper gastrointestinal dysfunction such as functional gastrointestinal disorders with reproducibility of lowering QOL of patients, particularly functional dyspepsia and gastroesophageal reflux disease.

The prophylactic or improving agent of the present invention contains a basic amino acid salt of glutamic acid, as an active ingredient.

The basic amino acid may be, for example, arginine, lysine or ornithine. Of these, arginine may be preferable.

The present invention may be applicable to improvable specific indefinite complaints covered by the term 'functional gastrointestinal disorders' including representative upper gastrointestinal indefinite complaint such as nausea, vomiting, sickly feeling, heartburn, feeling of fullness in the abdomen, heavy stomach, belching, chest writhing, chest pain, gastric discomfort, anorexia and dysphagia; lower gastrointestinal indefinite complaint such as abdominal pain, constipation and diarrhea; and related complaint such as breathlessness, feeling of smothering, low incentive, pharyngeal obstruction/feeling of foreign substance ("baikakuki" in Chinese medicine), easy fatigability, stiff neck, myotonia, mouth dryness (dry mouth/thirst), tachypnea, burning sensation/cold sensation of extremities, difficulty in concentration, impatience, sleep disorder, headache, general malaise, palpitation, night sweat, anxiety, dizziness, vertigo, burning sensation, hot flush, sweating, abdominal pain, constipation and depression.

The present invention, as mentioned above, enables prophylaxis or improvement of functional gastrointestinal disorders by administering an effective amount of the above-mentioned active ingredient to a subject. In this case, the active ingredient can be administered orally, enterally or parenterally as it is or after mixing with a pharmaceutical carrier and in the form of a pharmaceutical preparation such as tablet (including sugar-coated tablet, film-coated tablet), pill, capsule, ampoule, divided powder, elixir, suspension, syrup, gum preparation, drop preparation, powder, injection, suppository or sustained-release preparation, in consideration of the amount of an active ingredient to be administered, and the condition of the administration subject (e.g., patient). As the administration method, oral administration is preferable, and a sustained-release drug is more preferable. As the sustained-release form, conventional sustained-release preparations such as gel-coated preparation, multi-coated preparation and the like, gum preparation, drop preparation and localized release agent (pyloric part rupture preparation) can be mentioned.

As used herein, the "subject of administration" includes individuals affected with functional gastrointestinal disorders (e.g., human, domestic animals and poultry such as bovine, horse, swine, sheep, dog, bird, and experimental animals such as mouse or rat), and individuals having a risk of being affected with a functional gastrointestinal disorder. The "effective amount" means an amount sufficient to afford a desired improvement effect. While the dose of the active ingredient varies depending on the sex, age and body weight of administration subject, diet, form of administration, condition of FD, the level of risk inducing FD, and the condition of organic disease of the gastrointestinal tract, for example, the daily dose of the active ingredient to an adult (body weight 60 kg) is preferably 0.01 - 20 g, more preferably 0.01 - 10 g, and still more preferably 0.1 - 10 g. Such dose can be administered at once or in several portions.

The aforementioned "pharmaceutical carrier" means one which is pharmaceutically acceptable and least causes pharmacological action in the body. As a pharmaceutical carrier for oral administration, binders such as gum tragacanth, gum arabic, cornstarch and gelatin; excipients such as dicalcium phosphate; disintegrants such as cornstarch, potato starch and alginic acid; lubricants such as magnesium stearate; sweetening agents such as sucrose; dyes; flavorings such as orange flavor; solvents such as water, ethanol and glycerol; nutrients such as protein, amino acid, vitamin, lipid and glucose; can be used as appropriate. Furthermore, as a pharmaceutical carrier, pharmaceutically acceptable antioxidants such as cysteine, glutathione, ascorbic acid, sodium metasulfite and sodium bisulfite, and acid neutralizers such as calcium carbonate, hydroxide aluminum gel and aluminum silicate can be used.

The aforementioned dosage forms of the pharmaceutical preparations and pharmaceutical carriers are well known to those of ordinary skill in the art and, for example, the dosage forms and pharmaceutical carriers described in Remington's Pharmaceutical Science, ed. 16 (1980) (Mack Publishing Company) can be used.

The present invention may be used in combination with other pharmaceutical agents, and as such pharmaceutical agents, for example, acid secretion inhibitors such as H2 receptor antagonist or proton pump inhibitor; motility function improvers such as 5-HT receptor agonist or D2 antagonist; antacid agents such as muscarine receptor antagonist, anti-gastrin drug or anticholinergic drug; mucous membrane protectors such as teprenone, plaunotol, ornoprostil, enprostil, misoprostol, rebamipide, sucralfate, polaprezinc, azulene, egualen sodium, glutamine, aldioxa, gefarnate or ecabet sodium; inflammatory colitis treating agents such as sulfasalazine, 5-ASA preparation, steroid or remicade, can be contained. One or more kinds of these can be contained.

The food of the present invention is now explained. The food of the present invention comprises a basic amino acid salt of glutamic acid.

The food of the present invention may be prepared into a common food including what is called a health food. In addition, the food of the present invention may be prepared into a food with health claims, Food for specified health uses, Food with nutrient function claims, and further, a dietary supplement as defined by the food with health claims system of the Ministry of Health, Labour and Welfare.

As the food of the present invention, the aforementioned compound may be taken as it is. For easy intake, however, general food materials, seasonings and flavoring agents may be added to the above-mentioned compound. The mixture may be processed into a drink, gum, powder, tablet, granule, or jelly before intake. In this case, for example, a tablet made of the above-mentioned compound and a disintegrant, a mixture of the above-mentioned compound and a weighting agent (protein hydrolysate, starch, casein, glucose etc.), a mixture of the above-mentioned compound and an adhesive (gum, sublingual tablet, troche) which permits intraoral sustained-release, a solution of the above-mentioned compound in a solvent capable of dissolving the compound (e.g., edible fat and oil, ethanol, water), a W/O or O/W emulsion containing the above-mentioned compound, or a mixture of the above-mentioned compound and a nutrient (e.g., protein, amino acid, vitamin, lipid, glucose etc.) can be afforded. In addition, the agent of the invention can also be taken with a meal by addition thereto during the meal. For example, it can be taken by addition to an existing food such as drink, soft drink, yogurt, jelly or milk drink.

When the food of the present invention is used for the aforementioned particular object, the amount of intake of the glutamic acid salt per day for an adult is preferably 0.01 - 20 g, more preferably 0.01 - 10 g, and still more preferably 0.1 - 10 g. The content of the above-mentioned compound in the food of the present invention is generally 0.001 - 20 wt%, preferably 0.001 - 10 wt%, more preferably 0.01 - 10 wt%, still more preferably 0.1 - 10 wt%. By setting the content of the above-mentioned compound in the above-mentioned common food to fall within the above-mentioned range, a remarkable effect of improving gastrointestinal function can be afforded.

### Examples

The present invention is more specifically explained in the following by referring to Examples, which are not to be construed as limitative.

### (Example 1)

To study promotion of stomach emptying by monosodium glutamate and other salts, the following experiment was performed.

Male ICR mice were used. A 5% casein fluid diet (0.5 mL) containing 0.05% phenol red and a test drug was orally administered, and 30 min later, the chest was opened and the stomach was isolated. The stomach was placed in 0.1N sodium hydroxide (14 mL), homogenized and left standing for 1 hr at room temperature. 20% Trichloroacetic acid (0.5 mL) was added to 5 mL of the supernatant and the mixture was centrifuged (3000 rpm, 20 min). 0.5N sodium hydroxide (4 mL) was added to the supernatant and the absorbance was measured with an absorption spectrometer (560 nm). The gastric emptying rate was determined by the following calculation formula.

Gastric emptying rate (%) = (1- absorbance of test sample/absorbance of standard sample)×100 For absorbance of standard sample, the stomach isolated immediately after administration of 0.5% phenol red solution was used.

The test was performed using, after one-way analysis of variance, Dunnett's multiple comparison. **P*<0.05, ***P*<0.01, ****P*<0.001.

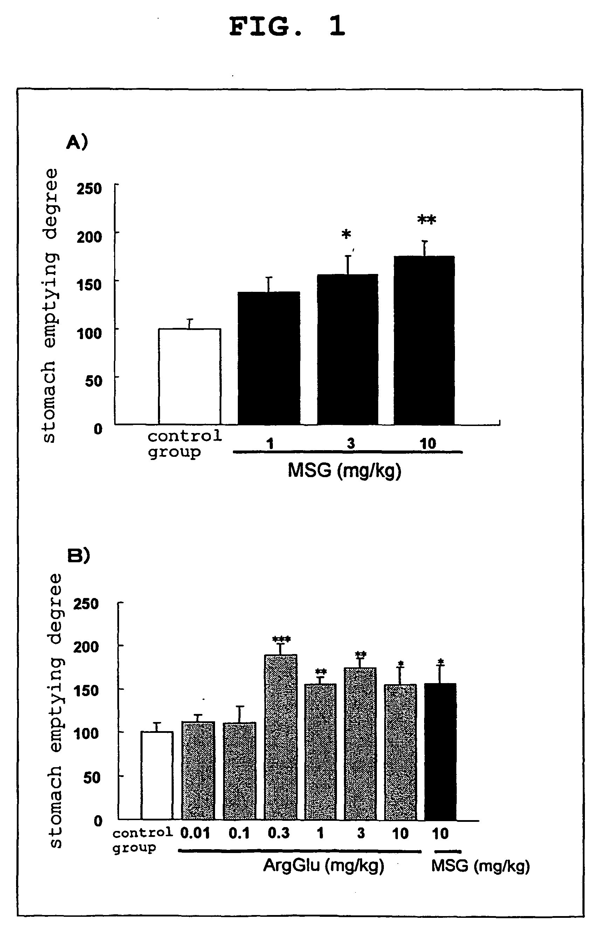

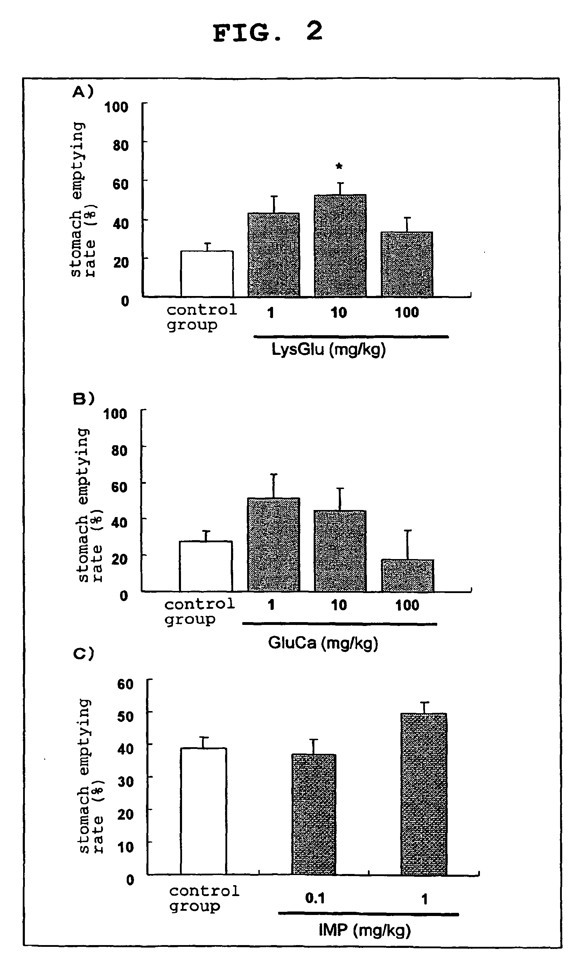

The results are shown in Figures. The number of cases in each group was 8-24. The vertical axis in Fig. 2 shows a stomach emptying rate (Figs. 2A and 2B), and Figs. 1A and 1B show stomach emptying degrees, with the stomach emptying rate of the control group as 100. Monosodium glutamate and arginine glutamic acid salt promoted stomach emptying (Figs. 1A, 1B). By comparison of the effects, it has been clarified that arginine salt promotes stomach emptying from lower doses as compared to sodium salt. In addition, lysine glutamic acid salt and calcium glutamate were similarly studied. As a result, it has been clarified that they promote stomach emptying (Figs. 2A, 2B). Based thereon, glutamic acid has been clarified to promote stomach emptying even when it is in other salt form. Moreover, inosinic acid was also studied and found to promote stomach emptying (Fig. 27C).

### Industrial Applicability

According to the present invention, a pharmaceutical agent and a food useful for the improvement of functional gastrointestinal disorders, particularly upper gastrointestinal dysfunction such as functional dyspepsia and gastroesophageal reflux disease can be provided. Since the concentration of NO and/or serotonin can be increased only in the gastrointestinal tract by the administration of the pharmaceutical agent of the present invention to an administration subject, gastrointestinal motility function can be effectively enhanced. Therefore, indefinite complaint accompanying gastrointestinal dysfunction such as FD can be improved safely and effectively without inducing a systemic side effect heretofore concerned.

## Claims

1. An agent for use in the prophylaxis or improvement of a "functional gastrointestinal disorder, which comprises a basic amino acid salt of glutamic acid as an active ingredient.

2. The agent of claim 1, wherein the basic amino acid is selected from the group consisting of arginine, lysine and ornithine.

3. The agent of claim 1, wherein the basic amino acid is arginine.

4. An agent for use in the prophylaxis or improvement of a functional gastrointestinal disorder, which comprises an arginine salt of glutamic acid as an active ingredient.

5. The agent of any one of claims 1 to 4, wherein said functional gastrointestinal disorder is upper gastrointestinal dysfunction.

6. The agent of claim 5, wherein said upper gastrointestinal dysfunction is functional dyspepsia or gastroesophageal reflux disease.

7. The agent of any one of claims 1 to 6, wherein a daily dose of the active ingredient to an adult is 0.01 g to 20 g.

8. An agent for use in promoting gastrointestinal motility function, which comprises a basic amino acid salt of glutamic acid as an active ingredient.

9. An agent for use in the prophylaxis or improvement of dysphagia, which comprises a basic amino acid salt of glutamic acid as an active ingredient.

10. A food for use in promoting gastrointestinal motility function, which comprises a basic amino acid salt of glutamic acid.

11. A food for use in the prophylaxis or improvement of dysphagia, which comprises a basic amino acid salt of glutamic acid.

12. An agent according to any one of claims 1 to 7, which is a food.

13. The food of any one of claims 10 to 12, wherein the content of said basic amino acid salt of glutamic acid is 0.01 wt% to 10 wt%.

14. The food of claim 13, which is a food with health claims or a dietary supplement.

15. The food of claim 14, wherein said food with health claims is a food for specified health uses or a food with nutrient function claims.

16. A commercial package comprising a composition comprising a basic amino acid salt of glutamic acid, and a written matter stating that the composition can or should be used for at least one kind selected from prophylaxis or improvement of a functional gastrointestinal disorder, promotion of gastrointestinal motility function and prophylaxis or improvement of dysphagia.

17. A commercial package according to claim 16 wherein the composition is a food.

18. Use of a basic amino acid salt glutamic acid for the production of an agent, food, or commercial package, according to any one of claims 1 to 17.

## Patentansprüche

1. Mittel für die Verwendung in der Prophylaxe oder Verbesserung einer funktionalen gastrointestinalen Störung, welches ein basisches Aminosäuresalz von Glutaminsäure als aktiven Bestandteil enthält.

2. Mittel nach Anspruch 1, wobei die basische Aminosäure aus der aus Arginin, Lysis und Ornithin bestehenden Gruppe ausgewählt ist.

3. Mittel nach Anspruch 1, wobei die basische Aminosäure Arginin ist.

4. Mittel zur Verwendung in der Prophylaxe oder Verbesserung einer funktionalen gastrointestinalen Störung, welches ein Argininsalz von Glutaminsäure als aktiven Bestandteil enthält.

5. Mittel nach einem der Ansprüche 1 bis 4, wobei die funktionelle gastrointestinale Störung eine Fehlfunktion des oberen Gastrointestinalbereichs ist.

6. Mittel nach Anspruch 5, wobei die Fehlfunktion des oberen Gastrointestinalbereichs funktionale Verdauungsstörungen oder gastroösophage Refluxbeschwerden sind.

7. Mittel nach einem der Ansprüche 1 bis 6, wobei eine tägliche Dosis des an einen Erwachsenen verabreichten aktiven Bestandteils 0,01 g bis 20 g ist.

8. Mittel zur Verwendung in der Forderung der gastrointestinalen Motilitätsfunktion, welches ein basisches Aminosäuresalz von Glutaminsäure als aktiven Bestandteil enthält.

9. Mittel zur Verwendung in der Prophylaxe oder Verbesserung von Dysphagie, welches ein basisches Aminosäuresalz von Glutaminsäure als aktiven Bestandteil enthält.

10. Nahrungsmittel für die Verwendung in der Förderung der gastrointestinalen Motilitätsfunktion, welches ein basisches Aminosäuresalz von Glutaminsäure enthält.

11. Nahrungsmittel für die Verwendung in der Prophylaxe oder Verbesserung von Dysphagie, welches ein basisches Aminosäuresalz von Glutaminsäure enthält.

12. Mittel nach einem der Ansprüche 1 bis 7, welches ein Nahrungsmittel ist.

13. Nahrungsmittel nach einem der Ansprüche 10 bis 12, wobei der Gehalt des basischen Aminosäuresalzes von Glutaminsäure 0,01 bis 10 Gew.-% ist.

14. Nahrungsmittel nach Anspruch 13, das ein gesundheitsbezogenes Nahrungsmittel oder ein diätetisches Zusatz ist.

15. Nahrungsmittel nach Anspruch 14, wobei das gesundheitsbezogene Nahrungsmittel ein Nahrungsmittel für bestimmte Gesundheitsanwendungen oder ein Nahrungsmittel mit Nährstofffunktionsangaben ist.

16. Kommerzielle Packung, die eine Zusammensetzung, welche ein basisches Aminosäuresalz von Glutaminsäure enthält, und eine schriftliche Anweisung umfasst, dass die Zusaminensetzung für mindestens eine Art von Anwendung verwendet werden kann oder soll, welche ausgewählt ist unter der Prophylaxe oder Verbesserung einer funktionalen gastrointestinalen Störung, der Förderung der gastrointestinalen Motilitätsfunktion und der Prophylaxe oder Verbesserung von Dysphagie.

17. Kommerzielle Packung nach Anspruch 16, wo die Zusammensetzung ein Nahrungsmittel ist.

18. Verwendung eines basischen Aminosäuresalzes von Glutaminsäure für die Herstellung eines Mittels, Nahrungsmitteils oder einer kommerziellen Packung nach einem der Ansprüche 1 bis 17.

## Revendications

1. Agent pour son utilisation dans la prophylaxie ou l'amélioration d'un trouble gastro-intestinal fonctionnel, qui comprend un sel d'acide aminé basique de l'acide glutamique en tant que principe actif.

2. Agent selon la revendication 1, où l'acide aminé basique est choisi dans le groupe constitué par l'arginine, la lysine et l'ornithine.

3. Agent selon la revendication 1, où l'acide aminé basique est l'arginine.

4. Agent pour son utilisation dans la prophylaxie ou l'amélioration d'un trouble gastro-intestinal fonctionnel, qui comprend un sel d'arginine de l'acide glutamique en tant que principe actif.

5. Agent selon l'une quelconque des revendications 1 à 4, où ledit trouble gastrointestinal fonctionnel est un dysfonctionnement gastro-intestinal supérieur.

6. Agent selon la revendication 5, où ledit dysfonctionnement gastro-intestinal supérieur est une dyspepsie fonctionnelle ou un reflux gastro-oesophagien pathologique.

7. Agent selon l'une quelconque des revendications 1 à 6, où une dose quotidienne du principe actif à un adulte est de 0,01 g à 20 g.

8. Agent pour son utilisation dans l'amélioration de la fonction de la motilité gastro-intestinale, qui comprend un sel d'acide aminé basique de l'acide glutamique en tant que principe actif.

9. Agent pour son utilisation dans la prophylaxie ou l'amélioration d'une dysphagie, qui comprend un sel d'acide aminé basique de l'acide glutamique en tant que principe actif.

10. Aliment pour son utilisation dans l'activation de la fonction de la motilité gastro-intestinale, qui comprend un sel d'acide aminé basique de l'acide glutamique en tant que principe actif.

11. Aliment pour son utilisation dans la prophylaxie ou l'amélioration d'une dysphagie, qui comprend un sel d'acide aminé basique de l'acide glutamique en tant que principe actif.

12. Agent selon l'une quelconque des revendications 1 à 7, qui est un aliment.

13. Aliment selon l'une quelconque des revendications 10 à 12, où la teneur en ledit sel d'acide aminé basique de l'acide glutamique est de 0,01 % en poids à 10 % en poids.

14. Aliment selon la revendication 13, qui est un aliment ayant des allégations de santé ou un complément alimentaire.

15. Aliment selon la revendication 14, où ledit aliment ayant des allégations de santé est un aliment pour des utilisations sanitaires spécifiques ou un aliment ayant des allégations de fonction nutritive.

16. Conditionnement commercial comprenant une composition comprenant un sel d'acide aminé basique de l'acide glutamique et un imprimé spécifiant que la composition peut ou doit être utilisée pour au moins une propriété choisie parmi la prophylaxie ou l'amélioration d'un trouble gastro-intestinal fonctionnel, l'activation de la fonction de la motilité gastro-intestinale et la prophylaxie ou l'amélioration de la dysphagie.

17. Conditionnement commercial selon la revendication 16, où la composition est un aliment.

18. Utilisation d'un sel d'acide aminé basique de l'acide glutamique pour la production d'un agent, d'un aliment ou d'un conditionnement commercial, selon l'une quelconque des revendications 1 à 17.
